# EUROPEAN PATENT APPLICATION

(11) **EP 3 792 633 A1**
(43) Date of publication of application: **17.03.2021**
(21) Application number: 20746837.2
(22) Date of filing: 09.01.2020
(51) Int. Cl.: G01N 33/76

(54) **HUMAN CHORIONIC GONADOTROPIN SEMI-QUANTITATIVE DETECTION TEST PAPER AND PREPARATION METHOD THEREFOR, HUMAN CHORIONIC GONADOTROPIN SEMI-QUANTITATIVE DETECTION REAGENT CUP, AND APPLICATIONS THEREOF**

(30) Priority: 30.07.2019 CN 201910693957
(71) Applicant: Nantong Egens Biotechnology Co., Ltd., Nantong, Jiangsu 226010 (CN)
(72) Inventor: The designation of the inventor has not yet been filed
(74) Representative: Lavoix
(86) International application number: PCT/CN2020/071034
(87) International publication number: WO 2021/017417

(57) **Abstract**

A test paper for semi-quantitative detection of human chorionic gonadotropin and preparation method, a reagent cup comprising the test paper, and a use of the test paper and the reagent cup for detecting human pregnancy cycle, ectopic pregnancy, miscarriage and trophoblastic disease. The test paper uses non-murine free β antibody to make the concentration detection range wider. Moreover, the test paper is provided with at least three test paper strips, which can be used to determine whether the detection line of each test paper strip develops color, a semi-quantitative identification of the concentration in a wide range can be made without subjective identification of color depth, thus the identified concentration range is more accurate. The reagent cup has an easy to use and hygienic, and suitable for on-site, instant and rapid.

## Description

### CROSS REFERENCE TO RELATED APPLICATIONS

This application claims priority to Chinese Patent Application No. 201910693957.1 filed on July 30, 2019, entitled "Test paper for semi-quantitative detection of human chorionic gonadotropin, reagent cup, preparation method therefor and use thereof', the entire contents of which are incorporated herein by reference.

### TECHNICAL FIELD

The present application relates to a field of biological detection, and particularly relates to a test paper for semi-quantitative detection of human chorionic gonadotropin, reagent cup, preparation method therefor and use thereof.

### BACKGROUND

Human chorionic gonadotropin (hCG) is a glycoprotein hormone secreted by placental trophoblast cells during pregnancy and consists of two different subunits of α and β connected by a non-covalent bond. During production, secretion, and metabolism of hormones, hCG molecules will undergo various changes such as rupture and dissociation, so as to exist in various molecular forms in blood and urine. hCG is the only placenta hormone that does not increase with the increase in placental weight. Reference values of hCG of pregnant women are described in "*Chinese Clinical Test Operating Procedures*": the reference value of hCG is 5-50 IU/ml for 0.2-1 week of pregnancy, the reference value of hCG is 50-500 IU/ml for 1-2 weeks of pregnancy, the reference value of hCG is 100-5000 IU/ml for 2-3 weeks of pregnancy, the reference value of hCG is 500-10000 IU/ml for 3-4 weeks of pregnancy, the reference value of hCG is 1000-50,000 IU/ml for 4-5 weeks of pregnancy, the reference value of hCG is 10,000-100,000 IU/ml for 5-6 weeks of pregnancy, the reference value of hCG is 15,000-200,000 IU/m for 6-8 weeks of pregnancy, the reference value of hCG is 10,000-100,000 IU/ml for 8-12 weeks of pregnancy, and decreases rapidly after the next 1-2 weeks, and then gradually decreases and maintains at about 1/5-1/10 of the peak level until delivery. Therefore, the pregnancy cycle can be estimated by detecting the concentration of hCG in blood and urine.

Moreover, the detection of human chorionic gonadotropin (hCG) has also been widely used in the diagnosis and observation of the effect of diseases involving threatened abortion, inevitable abortion, missed abortion, ectopic pregnancy, trophoblastic disease, etc. By detecting the concentration of hCG, various diseases can be diagnosed, and the course of the disease can be observed, which has great clinical significance.

At present, colloidal gold early pregnancy test paper is commonly used for hCG detection, but it can only make qualitative identification, and its application range is limited.

Chinese patent CN108761099A discloses a hCG cycle test paper comprising a first test paper strip and a second test paper strip. Whether the pregnancy time is the first three weeks or more than three weeks can be identified by comparing the degree of color development of detection lines of two test paper strips. The patent also discloses a hCG cycle test kit comprising the hCG cycle test paper. The test paper and the test kit have a simple operation method, and thus are suitable for on-site, instant, and rapid detection and are suitable for the detection of the number of weeks of pregnancy in a family. However, the test paper strip only comprises two test paper strips, including the first test paper strip with a minimum detectable quantity of 25mIU/ml and the second test paper strip with a minimum detectable quantity of 100mIU/ml, and can only determine whether the pregnancy time is the first three weeks or more than three weeks. When the person is pregnant for a longer period of time and has a hCG concentration of more than 100mIU/ml, the quality control line is easy to fail to develop color because the free β antibody used in the second test paper strip is a murine antibody. Therefore, it is impossible to identify the higher concentration range of hCG, and impossible to diagnose and identify various diseases, and the concentration detection range is narrow. Moreover, the identification using the test paper strip is performed by comparing the degree of color development of detection lines of two test paper strips, thus the test results are subjective and not accurate enough.

### SUMMARY

Base on the above, the technical problem to be solved by the present application is to overcome the defects that concentration detection range of the hCG detection test paper in the prior art is narrow, and the test results are subjective and not accurate enough. Therefore, the present application provides a test paper for semi-quantitative detection of human chorionic gonadotropin with a wide concentration detection range, accurate test results, and high sensitivity, reagent cup, preparation method therefor and use thereof.

In the first aspect, the present application provides a test paper for semi-quantitative detection of human chorionic gonadotropin, comprising at least three test paper strips, each including a substrate, and a sample pad, a colloidal gold adsorption pad, an antibody carrying film and a water absorption pad sequentially adhered to the substrate; wherein the sample pad, the colloidal gold adsorption pad, the antibody carrying film, and the water absorption pad are partially overlapped and lapped with each other;
the antibody carrying film is provided with a detection line on an end close to the colloidal gold adsorption pad and a quality control line on an end close to the water adsorption pad; the detection line is coated with an anti-human chorionic gonadotropin α-hCG monoclonal antibody; the quality control line is coated with an anti-mouse IgG polyclonal antibody;
the colloidal gold adsorption pad of one of the at least three test paper strips is adsorbed with colloidal gold-anti-human chorionic gonadotropin β-hCG monoclonal antibody conjugate, and the colloidal gold adsorption pad of the rest of the at least three test paper strips is adsorbed with colloidal gold-anti-human chorionic gonadotropin β-hCG monoclonal antibody conjugate and a free β antibody with different weight ratio; wherein the free β antibody is a non-murine antibody.

In the test paper for semi-quantitative detection of human chorionic gonadotropin, the free β antibody is a humanized, equine, rabbit or goat antibody.

The test paper for semi-quantitative detection of human chorionic gonadotropin comprises six test paper strips of a first test paper strip, a second test paper strip, a third test paper strip, a fourth test paper strip, a fifth test paper strip and a sixth test paper strip.

In the test paper for semi-quantitative detection of human chorionic gonadotropin, the weight ratios of colloidal gold-anti-human chorionic gonadotropin β-hCG monoclonal antibody conjugate to free β antibody adsorbed on colloidal gold adsorption pad of the second test paper strip, the third test paper strip, the fourth test paper strip, the fifth test paper strip, and the sixth test paper strip are 1:1, 1:2, 1:40, 1:80, 1:100, respectively.

In the test paper for semi-quantitative detection of human chorionic gonadotropin, the antibody carrying film is a nitrocellulose membrane with a pore size of 3-10 µm.

In the test paper for semi-quantitative detection of human chorionic gonadotropin, the interval between the detection line and the quality control line is 0.3-1.0 cm, preferably, 0.5 cm.

In the test paper for semi-quantitative detection of human chorionic gonadotropin, a protective film is disposed on both of the sample pad and the water adsorption pad.

In the second aspect, the present application provides a method for preparing a test paper for semi-quantitative detection of human chorionic gonadotropin, comprising the steps of:
S1: coating an antibody carrying film with anti-human chorionic gonadotropin α-hCG monoclonal antibody and anti-mouse IgG polyclonal antibody, respectively, to obtain a detection line and a quality control line, performing sealing treatment in a sealing treatment solution, and drying for use;
S2: taking colloidal gold and adjusting pH thereof, adding anti-human chorionic gonadotropin β-hCG monoclonal antibody therein, adding a stabilizer with stirring, centrifuging and collecting a precipitate, redissolving the precipitate with a colloidal gold complex solution to obtain colloidal gold-anti-human chorionic gonadotropin β-hCG monoclonal antibody conjugate complex solution;
S3: casting the colloidal gold-anti-human chorionic gonadotropin β-hCG monoclonal antibody conjugate complex solution obtained in step S2 on a colloidal gold adsorption pad of one test paper strip, followed by drying, sealing and storing for use;
S4: adding free β antibody to the colloidal gold-anti-human chorionic gonadotropin β-hCG monoclonal antibody conjugate complex solution obtained in step S2, and mixing well to obtain different mixed complex solutions with different weight ratio of colloidal gold-anti-human chorionic gonadotropin β-hCG monoclonal antibody conjugate to free β antibody, casting the different mixed complex solutions on colloidal gold adsorption pads of the rest of test paper strips, followed by drying, sealing and storing for use;
S5: taking a sample pad, a colloidal gold adsorption pad, an antibody carrying film, and a water adsorption pad which are pasted sequentially along a length direction of a substrate in a partially overlapping manner, and obtaining a test paper strip, and preparing at least three test paper strips in this way;
wherein the free β antibody is a non-murine antibody.

In the method for preparing a test paper for semi-quantitative detection of human chorionic gonadotropin, there are six test paper strips of a first test paper strip, a second test paper strip, a third test paper strip, a fourth test paper strip, a fifth test paper strip and a sixth test paper strip.

In the method for preparing a test paper for semi-quantitative detection of human chorionic gonadotropin, in the first, second, and third test paper strips, the anti-human chorionic gonadotropin β -hCG monoclonal antibody is labeled with colloidal gold at 4 µg/ml, and in the fourth, fifth, and sixth test paper strips, the anti-human chorionic gonadotropin β-hCG monoclonal antibody is labeled with colloidal gold at 5 µg/ml.

In the method for preparing a test paper for semi-quantitative detection of human chorionic gonadotropin, in the second, third, fourth, fifth, and sixth test paper strips, the weight ratios of the anti-human chorionic gonadotropin β-hCG monoclonal antibody to the free β antibody are 1:1, 1:2, 1:40, 1:80, and 1:100, respectively.

In the method for preparing a test paper for semi-quantitative detection of human chorionic gonadotropin, the anti-human chorionic gonadotropin α-hCG monoclonal antibody has a concentration of 2-3 mg/ml, the anti-mouse IgG polyclonal antibody has a concentration of 1-2 mg/ml.

In the method for preparing a test paper for semi-quantitative detection of human chorionic gonadotropin, the sealing treatment solution comprises 0.08-0.12Mol buffer solution, 0.3-0.7wt% sugar, and 0.8-1.2wt% sealing protein, and 0.03-0.07wt% preservative, wherein the buffer solution is phosphate buffer, the sugar is sucrose or trehalose, the preservative is NaN₃ or thimerosal, and the blocking protein is casein or bovine serum albumin. Preferably, the sealing treatment solution comprises 0.1Mol buffer solution, 0.5wt% sugar, 1wt% sealing protein, and 0.05wt% preservative.

In the method for preparing a test paper for semi-quantitative detection of human chorionic gonadotropin, in step S2, the pH is adjusted to 6.5-7.0.

In the method for preparing a test paper for semi-quantitative detection of human chorionic gonadotropin, the colloidal gold-anti-human chorionic gonadotropin β-hCG monoclonal antibody conjugate complex solution and the mixture solution of colloidal gold-anti-human chorionic gonadotropin β-hCG monoclonal antibody conjugate and free β antibody are cast on the colloidal gold adsorption pad with an amount of 50 µl/cm² respectively.

In the third aspect, the present application provides a reagent cup for semi-quantitative detection of human chorionic gonadotropin, comprising the test paper for semi-quantitative detection of human chorionic gonadotropin or the test paper for semi-quantitative detection of human chorionic gonadotropin prepared by the method.

The reagent cup for semi-quantitative detection of human chorionic gonadotropin further comprises a cup body, and a cartridge disposed inside the reagent cup and provided with parallel slots, wherein, one end of the slot is provided with an opening for the test paper strip to be inserted therein, a sample pad of a test paper strip extends from the opening to an outside of the cartridge.

The reagent cup for semi-quantitative detection of human chorionic gonadotropin further comprises a cup cover detachably closed on the cup body.

In the reagent cup for semi-quantitative detection of human chorionic gonadotropin, the cartridge is disposed vertically inside the cup body, and the opening of the slot is close to the bottom of the cup body.

In the reagent cup for semi-quantitative detection of human chorionic gonadotropin, the cup body is provided with a detection cavity, a liquid storage cavity and a liquid guide cavity; wherein the liquid guide cavity is provided with a first through hole intercommunicating with the liquid storage cavity at an upper part thereof and a second through hole intercommunicating with the detection cavity at a lower part thereof; a piston sliding along the liquid guide cavity is disposed inside the liquid guide cavity, and has an initial state for intercommunicating the liquid guide cavity with the liquid storage cavity and a detection state for intercommunicating the liquid chamber guide cavity with the detection chamber; a third through hole is disposed on a cup wall of the cup body away from the detection chamber and intercommunicated with the liquid guide cavity; and the cartridge is disposed vertically inside the detection cavity.

In the reagent cup for semi-quantitative detection of human chorionic gonadotropin, a liquid storage tank is disposed on the piston; when the piston is in the initial state, the liquid storage tank of the piston is aligned with the first through hole, so that the liquid guide cavity is intercommunicated with the liquid storage cavity; when the piston is in the detection state, the liquid storage tank of the piston is aligned with the second through hole, so that the liquid guide cavity is intercommunicated with the detection cavity.

In the reagent cup for semi-quantitative detection of human chorionic gonadotropin, a boost member for boosting the piston is provided on the cup cover, and detachably fitted on the cup cover.

In the reagent cup for semi-quantitative detection of human chorionic gonadotropin, the cartridge is fixed in parallel inside the cup cover.

In the reagent cup for semi-quantitative detection of human chorionic gonadotropin, a support body is disposed on an outer edge of the cup cover for fixing the reagent cup placed on its side, and a scale line is arranged on the cup body.

In the reagent cup for semi-quantitative detection of human chorionic gonadotropin, the cartridge is made of transparent material.

In the fourth aspect, the present application provides a method for semi-quantitative detection of human chorionic gonadotropin using the reagent cup, which comprises collecting a urine sample in the cup body, and observing whether the detection line and the quality control line of the test paper strip develop color within 5 minutes from the time when the test paper strip adsorbs the sample.

In the fifth aspect, the present application provides a use of a test paper for semi-quantitative detection of human chorionic gonadotropin, the test paper for semi-quantitative detection of human chorionic gonadotropin prepared by the method, or a reagent cup for semi-quantitative detection of human chorionic gonadotropin for detecting human pregnancy cycle, ectopic pregnancy, miscarriage and trophoblastic disease.

The technical solutions provided by the present application have the following advantages.
1. The test paper for semi-quantitative detection of human chorionic gonadotropin provided by the present application, comprises at least three test paper strips, each including a substrate, and a sample pad, a colloidal gold adsorption pad, an antibody carrying film and a water absorption pad sequentially adhered to the substrate; wherein the sample pad, the colloidal gold adsorption pad, the antibody carrying film, and the water absorption pad are partially overlapped and lapped with each other; the antibody carrying film is provided with a detection line on an end close to the colloidal gold adsorption pad and a quality control line on an end close to the water adsorption pad; the detection line is coated with an anti-human chorionic gonadotropin α-hCG monoclonal antibody; the quality control line is coated with an anti-mouse IgG polyclonal antibody; the colloidal gold adsorption pad of one of the at least three test paper strips is adsorbed with colloidal gold-anti-human chorionic gonadotropin β-hCG monoclonal antibody conjugate, and the colloidal gold adsorption pad of the rest of the at least three test paper strips is adsorbed with colloidal gold-anti-human chorionic gonadotropin β-hCG monoclonal antibody conjugate and a free β antibody with different weight ratio; wherein the free β antibody is a non-murine antibody. The test paper can adjust the detectable quantity of the test paper to a wider range by using a non-murine free β antibody. If a murine free β antibody is used, when the concentration of hCG in the testing sample is high, it needs to use a large amount of free β antibody to adjust the detectable quantity of the test paper strip to a higher level. However, since the free β antibody is a murine antibody, a large amount of free β antibody may bind with the anti-mouse IgG polyclonal antibody disposed on the quality control line, but only a very small amount of colloidal gold-labeled hCG may bind with the quality control line, thus the quality control line does not develop color, which makes it difficult to detect a higher concentration of hCG. The concentration detection range becomes wider by using a non-murine β antibody. In addition, the test paper is provided with at least three test paper strips, each has different types and concentrations of antibodies. When detecting the concentration of hCG, the color development of each test paper strip is different. By determining whether the detection line of each test paper strip develops color, a semi-quantitative identification of the concentration in a wider range can be made without subjective identification of color depth, thus the concentration range is identified more accurately.
2. In the method for preparing a test paper for semi-quantitative detection of human chorionic gonadotropin provided by the present application, a non-murine free β antibody and colloidal gold-anti-human chorionic gonadotropin β-hCG monoclonal antibody conjugate are mixed and cast into the colloidal gold adsorption pad, which can overcome the defect that when the murine free β antibody is used, the concentration of hCG in the testing sample is high and the concentration of the murine free β antibody is high, the quality control line does not develop color, which makes it difficult to detect a higher concentration of hCG. The concentration detection range becomes wider by using a non-murine β antibody. In addition, the test paper is provided with at least three test paper strips, each has different types and concentrations of antibodies. When detecting the concentration of hCG, the color development of each test paper strip is different. By determining whether the detection line of each test paper strip develops color, a semi-quantitative identification of the concentration in a wider range can be made without subjective identification of color depth, thus the concentration range is identified more accurately.
3. In the method for preparing a test paper for semi-quantitative detection of human chorionic gonadotropin provided by the present application, the weight ratios of colloidal gold-anti-human chorionic gonadotropin β-hCG monoclonal antibody conjugate to free β antibody adsorbed on colloidal gold adsorption pad of the second test paper strip, the third test paper strip, the fourth test paper strip, the fifth test paper strip, and the sixth test paper strip are 1:1, 1:2, 1:40, 1:80, 1:100, respectively. By setting the above ratio gradients, the test paper strips with the minimum detectable quantities of 5mIU/ml, 25mIU/ml, 100mIU/ml, 500mIU/ml, 2500mIU/ml, and 10000mIU/ml respectively can be prepared, so as to obtain semi-quantitative test paper with high sensitivity, wide concentration detection range, and accurate test results.
4. The reagent cup for semi-quantitative detection of human chorionic gonadotropin provided by the present application, comprises the test paper for semi-quantitative detection of human chorionic gonadotropin or the test paper for semi-quantitative detection of human chorionic gonadotropin prepared by the method. The reagent cup is used to detect the concentration of hCG, and the detection range is wider. Moreover, the reagent cup can be used to make a semi-quantitative identification of the concentration by determining whether the detection line of each test paper strip develops color, without subjective identification of color depth, thus the concentration range is identified more accurately.
5. The reagent cup for semi-quantitative detection of human chorionic gonadotropin provided by the present application, further comprises a cup body, and a cartridge disposed inside the reagent cup and provided with parallel slots, wherein, one end of the slot is provided with an opening for the test paper strip to be inserted therein, a sample pad of a test paper strip extends from the opening to an outside of the cartridge. The cartridge with a slot is configured to fix the test paper strip in the reagent cup, which is convenient for sucking the sample. The sample pad of the test paper strip extends from the opening to the outside of the cartridge, it is convenient for the sample pad to suck the sample. When using the reagent cup, as long as the urine sample is placed in the cup body, the test can be completed, which is easy to use and hygienic.
6. The reagent cup for semi-quantitative detection of human chorionic gonadotropin provided by the present application further comprises a cup cover detachably closed on the cup body. During the test, the cup cover is covered after putting the urine sample into the cup body, on the one hand, which can isolate the sample from the external environment, ensuring the stability of the test environment, and preventing the external environment from affecting the test effect, and on the other hand, can prevent the emission of odor and make it more hygienic.
7. In the reagent cup for semi-quantitative detection of human chorionic gonadotropin provided in the present application, the cup body is provided with a detection cavity, a liquid storage cavity and a liquid guide cavity; wherein the liquid guide cavity is provided with a first through hole intercommunicating with the liquid storage cavity at an upper part thereof and a second through hole intercommunicating with the detection cavity at a lower part thereof; a piston sliding along the liquid guide cavity is disposed inside the liquid guide cavity, and has an initial state for intercommunicating the liquid guide cavity with the liquid storage cavity and a detection state for intercommunicating the liquid chamber guide cavity with the detection chamber; a third through hole is disposed on a cup wall of the cup body away from the detection chamber and intercommunicated with the liquid guide cavity; and the cartridge is disposed vertically inside the detection cavity. The detection cavity comprising the cartridge and the test paper, and a liquid storage cavity for collecting urine, are configured to make the test paper in the detection cavity separate from the urine sample under the initial state. The piston is configured to make the liquid storage cavity communicate with the liquid guide cavity under the initial state, and the urine enters into the liquid guide cavity from the liquid storage cavity. When the piston slides in the liquid guide cavity to the detection state, the urine enters into the detection cavity from the liquid guide cavity, thus the test paper strip in the cartridge of the detection cavity contacts with the urine sample, thus completing the detection. Through the above structure, the time when starts the test can be more conveniently controlled, and the test can be started after the sample collection is completed, so as to prevent the problem that the test result is not accurate enough when collecting samples while simultaneously performing the test, making the result more accurate and easy to operate. The liquid guide cavity intercommunicates with the third through hole, so that the sliding of the piston can be easily controlled.
8. In the reagent cup for semi-quantitative detection of human chorionic gonadotropin provided by the present application, a liquid storage tank is disposed on the piston; when the piston is in the initial state, the liquid storage tank of the piston is aligned with the first through hole, so that the liquid guide cavity is intercommunicated with the liquid storage cavity; when the piston is in the detection state, the liquid storage tank of the piston is aligned with the second through hole, so that the liquid guide cavity is intercommunicated with the detection cavity. The liquid storage tank is configured to make the urine sample flow into the liquid storage tank from the liquid storage cavity under the initial state, the piston is pushed, so that the urine sample can flow into the detection cavity from the liquid storage tank under the detection state, and the test paper strip in the detection cavity can suck samples, thereby starting the detection. The liquid storage tank is configured to make the amount of urine samples introduced into the detection cavity constant, thereby preventing excessive urine samples from entering the detection cavity and affecting the test results. The accuracy of the test results is ensured
9. In the reagent cup for semi-quantitative detection of human chorionic gonadotropin provided by the present application, a boost member for boosting the piston is provided on the cup cover, and detachably fitted on the cup cover. The boost member is configured to more easily push the piston from the initial state to the detection state. When the reagent cup is not used, the boost member can be fitted on the cup cover to facilitate storage and prevent its loss.
10. In the reagent cup for semi-quantitative detection of human chorionic gonadotropin provided by the present application, the cartridge is fixed in parallel inside the cup cover. By fixing the cartridge on the cup cover, the test paper strip in the cartridge can be separated from the urine sample in the cup body when the test is not started. At the beginning of the test, the test can be started by tightening the cup cover, and placing the reagent cup on its side, so as to prevent the problem that the test result is not accurate enough when collecting samples while simultaneously performing the test, making the result more accurate and easy to operate.
11. In the reagent cup for semi-quantitative detection of human chorionic gonadotropin provided by the present application, a support body is disposed on an outer edge of the cup cover for fixing the reagent cup placed on its side, and a scale line is arranged on the cup body. The support body is configured to dispose on the edge of the cup cover, the reagent cup is fixed when it is placed on its side, preventing the reagent cup from rolling. When collecting urine, it is necessary to ensure that the urine exceeds the scale line, ensuring that the urine samples are sufficient when the reagent cup is placed on its side, so that the test paper strip can carry out adsorption detection successfully.
12. In the use of a test paper for semi-quantitative detection of human chorionic gonadotropin, the test paper for semi-quantitative detection of human chorionic gonadotropin prepared by the method, or a reagent cup for semi-quantitative detection of human chorionic gonadotropin for detecting human pregnancy cycle, ectopic pregnancy, miscarriage and trophoblastic disease provided by the present application, the above test paper or reagent cup can be used to conduct a semi-quantitative detection of the concentration of hCG in a wider range. The detection range is wide, the sensitivity is high and the result is accurate, so that human pregnancy cycle can be identified more accurately, and diseases such as ectopic pregnancy, miscarriage and trophoblastic lesions can be diagnosed and identified by the detected concentration range.

### DESCRIPTION OF THE DRAWING

In order to more clearly illustrate the technical solutions of the embodiments of the present application or the prior art, the drawings used in the embodiments of the present application or the prior art will be briefly described below. Obviously, the drawings in the following description are only some examples of the present application, and those skilled in the art can obtain other drawings based on these drawings without any creative efforts.
Figure 1 is a schematic view of a test paper for semi-quantitative detection of human chorionic gonadotropin in Examples 1-6 of the present application;
Figure 2 is a schematic view of a test paper and a cartridge in the reagent cup for semi-quantitative detection of human chorionic gonadotropin in Examples 4-6 of the present application;
Figure 3 is a schematic view of a reagent cup for semi-quantitative detection of human chorionic gonadotropin in Example 4 of the present application;
Figure 4 is a schematic view of a reagent cup for semi-quantitative detection of human chorionic gonadotropin in Example 5 of the present application;
Figure 5 is a schematic view of a reagent cup for semi-quantitative detection of human chorionic gonadotropin in an initial state of Example 5 of the present application;
Figure 6 is a schematic view of a reagent cup for semi-quantitative detection of human chorionic gonadotropin in a detection state in Example 5 of the present application; and
Figure 7 is a schematic view of a reagent cup for semi-quantitative detection of human chorionic gonadotropin in Example 6 of the present application;

In the figures, the reference numerals are:
1-substrate; 2-sample pad; 3-colloidal gold adsorption pad; 4-antibody carrying film; 5-water adsorption pad; 6-detection line; 7-quality control line; 8-protective film; 9-cup body; 10-cup cover; 11-first test paper strip; 12-second test paper strip; 13-third test paper strip; 14-fourth test paper strip; 15-fifth test paper strip; 16-sixth test paper strip; 17-cartridge; 18-slot; 19-detection cavity; 20-liquid storage cavity; 21-liquid guide cavity; 211-first through hole; 212-second through hole; 213-third through hole; 22-piston; 221-liquid storage tank; 23-boost member; 24-support body; 25- scale line; 26-liquid guide tank.

### DETAILED DESCRIPTION OF THE EMBODIMENTS

Humanized free β antibody from hamster, equine free β antibody from horse, rabbit free β antibody from rabbit, anti-human chorionic gonadotropin α-hCG monoclonal antibody, anti-mouse IgG polyclonal antibody, anti-human chorionic gonadotropin β-hCG monoclonal antibody used in the following examples are purchased from Hangzhou Zhengzhi Biotechnology Co., Ltd.

### Example 1

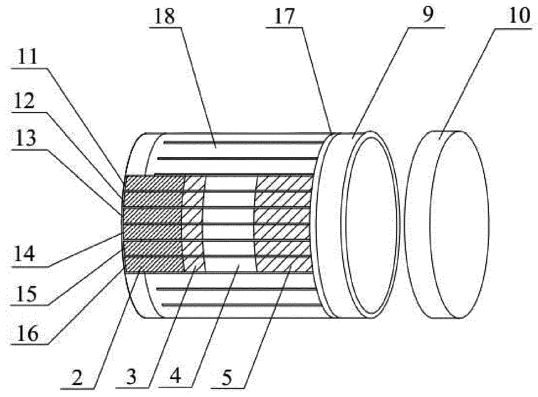

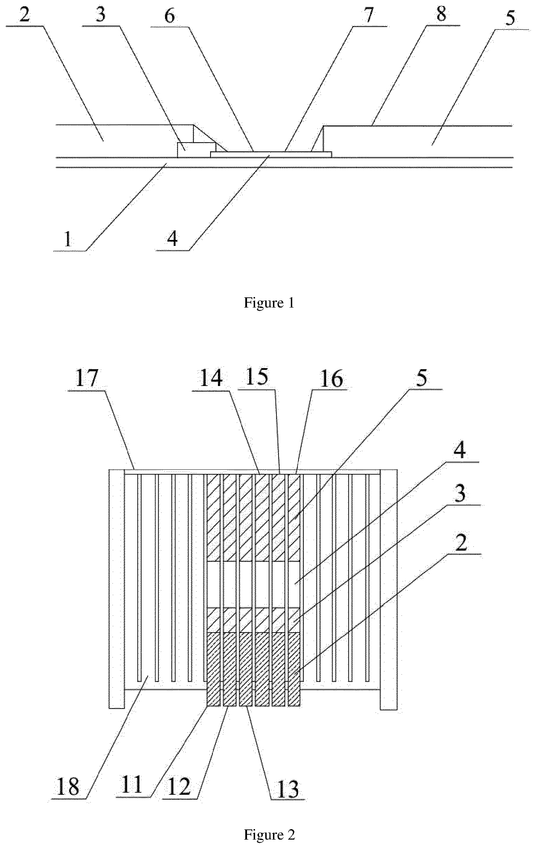

The present example provides a test paper for semi-quantitative detection of human chorionic gonadotropin, which comprises six test paper strips of a first test paper strip 11, a second test paper strip12, a third test paper strip13, a fourth test paper strip14, a fifth test paper strip 15 and a sixth test paper strip 16. The structure of each test paper strip, as shown in Figure 1, includes a substrate 1 and a sample pad 2, a colloidal gold adsorption pad 3, an antibody carrying film 4 and a water adsorption pad 5 sequentially adhered to the substrate. The sample pad 2, the colloidal gold adsorption pad 3, the antibody carrying film 4 and the water adsorption pad 5 are partially overlapped and lapped with each other. Specifically, the antibody carrying film 4 is located under the colloidal gold adsorption pad 3 and the water adsorption pad 5, the sample pad 2 is located above the colloidal gold adsorption pad 3, and the length of the overlapping part is 1mm.

The antibody carrying film 4 is provided with a detection line 6 on an end close to the colloidal gold adsorption pad 3 and a quality control line 7 on an end close to the water adsorption pad 5. The detection line 6 is coated with an anti-human chorionic gonadotropin α-hCG monoclonal antibody (anti-α-hCG monoclonal antibody). The quality control line 7 is coated with an anti-mouse IgG polyclonal antibody.

The colloidal gold adsorption pad 3 of the first test paper strip 11 is adsorbed with colloidal gold-anti-human chorionic gonadotropin β-hCG monoclonal antibody conjugate (colloidal gold-anti-β-hCG monoclonal antibody conjugate). The colloidal gold adsorption pad 3 of the second test paper strip 12, the third test paper strip 13, the fourth test paper strip 14, the fifth test paper strip 15 and the sixth test paper strip 16 is adsorbed with colloidal gold-anti-β-hCG monoclonal antibody conjugate and free β antibody. The weight ratio of gold-anti-β-hCG monoclonal antibody conjugate to free β antibody of the second test paper strip 12, the third test paper strip 13, the fourth test paper strip 14, the fifth test paper strip 15 and the sixth test paper strip 16 is 1:1, 1:2, 1:40, 1:80, and 1:100, respectively. The free β antibody is non-murine antibody. In the example, the free β antibody is a humanized antibody derived from hamster.

Moreover, the detection line 6 and the quality control line 7 are arranged in parallel with a pitch of 0.3-1.0 cm. In the example, the pitch is 0.5 cm.

Moreover, a protective film 8 is disposed on both of the sample pad 2 and the water adsorption pad 3.

Moreover, the antibody carrying film 4 is a nitrocellulose film with a pore size of 3-10 µm.

The present example provides a method for preparing the above test paper for semi-quantitative detection of human chorionic gonadotropin, comprises the following steps.
1. Preparation of an antibody carrying film:
   (1) A nitrocellulose film with a pore size of 3-10 µm is cut into a film having specifications with a width of 2.0 cm and a length of 30.5cm, as needed.
   (2) 2-3mg/ml anti-α-hCG monoclonal antibody is prepared with 0.1M phosphate buffer for coating of the detection line, in this example, the concentration of anti-α-hCG monoclonal antibody is 2.5 mg/ml. 1-2.0 mg/ml anti-mouse IgG polyclonal antibody is prepared with 0.85 wt% sodium chloride buffer for coating of the quality control line, in this example, the concentration of anti-mouse IgG polyclonal antibody is 1.5 mg/ml.
   (3) An antibody coating surface of the nitrocellulose film is labeled. The antibody solution of the detection line to be coated and the antibody solution of the quality control line to be coated should be uniformly coated on the film in parallel. The detection line and the quality control line are disposed at an interval of 0.3-0.7 cm. In this example, the interval is 0.5cm. The nitrocellulose film is dried at a constant temperature of 2-30 °C for use.
   (4) A sealing treatment soaking solution is prepared: an actual production volume of purified water is added to a mixing tank; and then buffer, sugar, sealing protein and preservative are respectively weighed and then directly added to a mixing tank with stirring until completely dissolved, purified water is added to reach a required volume, followed by stirring well for not less than 10 minutes. The sealing treatment soaking solution comprises 0.1Mol phosphate buffer, 0.5wt% sugar, 1wt% sealing protein and 0.05wt% preservative, wherein the buffer solution is phosphate buffer, the sugar is sucrose, the preservative is thimerosal, and the sealing protein is bovine serum albumin.
   (5) The film coated with the detection line and the quality control line is placed in a processing tank, and the sealing treatment soaking solution prepared in step (4) is added therein. It should be ensured that each film is completely immersed in the sealing treatment soaking solution for 30 minutes and the film does not move and overlap. The film is taken from the processing tank, and then the sealing treatment soaking solution is discarded. After that, the film is placed on a gauze with tweezers to dry it a little, so as to obtain an antibody carrying film.
   (6) Pasted on a board and drying

A white paper in the middle of a cutting line on a double-sided tape of a tape board is removed. An operator places the film in the blank space in the center of the tape board, and make sure that the right side of the tape board is flush with the right side of the film. In order to avoid errors in the production process, it is necessary to ensure that the color development position is relatively accurate. The film is pasted on the board by aligning with the top of one end of the quality control line. After the film is pasted on the tape board, the film surface is smoothed across double-sided tape to avoid air bubbles. The temperature in the room is controlled to 18-28 °C, and the relative humidity is ≤40%. It is also necessary to ensure that an air in a drying room could circulate and a wind of a dehumidifier will not directly blow on the film surface. The drying time is ≥4 hours.

### 2. Preparation of the colloidal gold adsorption pad of the first test paper strip

(1) Preparation of colloidal gold complex solution
   The actual production volume of purified water is added to the mixing tank; and then trehalose, bovine serum albumin, trisodium citrate, polyethylene glycol, and NaN₃ are weighed with an electronic analytical balance and then directly added to the mixing tank with stirring until completely dissolved, purified water is added to reach a required volume, followed by stirring well for not less than 30 minutes, thus preparing a colloidal gold complex solution containing 5wt% trehalose, 2wt% bovine serum albumin, 0.5wt% trisodium citrate, 0.05wt% polyethylene glycol, and 0.05wt% NaN₃.
(2) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-β-hCG monoclonal antibody is diluted with double distilled water, and labeled with the colloidal gold at 4 µg/ml, i.e. the anti-β-hCG monoclonal antibody is added into the colloidal gold, followed by stirring on a magnetic stirrer for 30 minutes, and then 0.5‰ by volume of stabilizer polyethylene glycol is added with stirring for 30 minutes, followed by centrifuging, collecting a labeled colloidal gold precipitate which is re-dissolved with the colloidal gold complex solution at 3% by volume, and stirring on a magnetic stirrer until the mixture is homogeneous, thus obtaining 3% by volume of complex solution of colloidal gold-anti-β-hCG monoclonal antibody conjugate.
(3) 3% by volume of the complex solution of colloidal gold-anti-β-hCG monoclonal antibody conjugate of the above step (2) is taken and re-dissolved with the colloidal gold complex solution at 50% by volume, followed by mixing on a magnetic stirrer, and then cast on the prepared colloidal gold adsorption pad at 50 µl/cm², followed by placing in a drying room to dry for> 4 hours, and controlling the temperature in the drying room at 18-28 °C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as the colloidal gold adsorption pad of the first test paper strip.

### 3. Preparation of the colloidal gold adsorption pad of the second test paper strip

(1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-β-hCG monoclonal antibody is diluted with double distilled water, and labeled with the colloidal gold at 4 µg/ml, i.e. the anti-β-hCG monoclonal antibody is added into the colloidal gold, followed by stirring on a magnetic stirrer for 30 minutes, and then 0.5‰ by volume of stabilizer polyethylene glycol is added with stirring for 30 minutes, followed by centrifuging, collecting a labeled colloidal gold precipitate which is re-dissolved with the colloidal gold complex solution at 3% by volume, and stirring on a magnetic stirrer until the mixture is homogeneous, thus obtaining 3% by volume of complex solution of colloidal gold-anti-β-hCG monoclonal antibody conjugate.
(2) 3% by volume of the complex solution of colloidal gold-anti-β-hCG monoclonal antibody conjugate of the above step (1) is taken and re-dissolved with the colloidal gold complex solution at 40% by volume, then added free β antibody according to the weight ratio of anti-β-hCG monoclonal antibody to free β antibody of 1:1, wherein the free β antibody is a humanized antibody derived from hamster, followed by mixing on a magnetic stirrer, and then cast on the prepared colloidal gold adsorption pad at 50 µl/cm², followed by placing in a drying room to dry for> 4 hours, and controlling the temperature in the drying room at 18-28 °C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as the colloidal gold adsorption pad of the second test paper strip.

### 4. Preparation of the colloidal gold adsorption pad of the third test paper strip

(1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-β-hCG monoclonal antibody is diluted with double distilled water, and labeled with the colloidal gold at 4 µg/ml, i.e. the anti-β-hCG monoclonal antibody is added into the colloidal gold, followed by stirring on a magnetic stirrer for 30 minutes, and then 0.5‰ by volume of stabilizer polyethylene glycol is added with stirring for 30 minutes, followed by centrifuging, collecting a labeled colloidal gold precipitate which is re-dissolved with the colloidal gold complex solution at 3% by volume, and stirring on a magnetic stirrer until the mixture is homogeneous, thus obtaining 3% by volume of complex solution of colloidal gold-anti-β-hCG monoclonal antibody conjugate.
(2) 3% by volume of the complex solution of colloidal gold-anti-β-hCG monoclonal antibody conjugate of the above step (1) is taken and re-dissolved with the colloidal gold complex solution at 40% by volume, then added free β antibody according to the weight ratio of anti-β-hCG monoclonal antibody to free β antibody of 1:2, wherein the free β antibody is a humanized antibody derived from hamster, followed by mixing on a magnetic stirrer, and then cast on the prepared colloidal gold adsorption pad at 50 µl/cm², followed by placing in a drying room to dry for> 4 hours, and controlling the temperature in the drying room at 18-28 °C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as the colloidal gold adsorption pad of the third test paper strip.

### 5. Preparation of the colloidal gold adsorption pad of the fourth test paper strip

(1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-β-hCG monoclonal antibody is diluted with double distilled water, and labeled with the colloidal gold at 5 µg/ml, i.e. the anti-β-hCG monoclonal antibody is added into the colloidal gold, followed by stirring on a magnetic stirrer for 30 minutes, and then 0.5‰ by volume of stabilizer polyethylene glycol is added with stirring for 30 minutes, followed by centrifuging, collecting a labeled colloidal gold precipitate which is re-dissolved with the colloidal gold complex solution at 3% by volume, and stirring on a magnetic stirrer until the mixture is homogeneous, thus obtaining 3% by volume of complex solution of colloidal gold-anti-β-hCG monoclonal antibody conjugate.
(2) 3% by volume of the complex solution of colloidal gold-anti-β-hCG monoclonal antibody conjugate of the above step (1) is taken and re-dissolved with the colloidal gold complex solution at 40% by volume, then added free β antibody according to the weight ratio of anti-β-hCG monoclonal antibody to free β antibody of 1:40, wherein the free β antibody is a humanized antibody derived from hamster, followed by mixing on a magnetic stirrer, and then cast on the prepared colloidal gold adsorption pad at 50 µl/cm², followed by placing in a drying room to dry for> 4 hours, and controlling the temperature in the drying room at 18-28 °C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as the colloidal gold adsorption pad of the fourth test paper strip.

### 6. Preparation of the colloidal gold adsorption pad of the fifth test paper strip

(1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-β-hCG monoclonal antibody is diluted with double distilled water, and labeled with the colloidal gold at 5 µg/ml, i.e. the anti-β-hCG monoclonal antibody is added into the colloidal gold, followed by stirring on a magnetic stirrer for 30 minutes, and then 0.5‰ by volume of stabilizer polyethylene glycol is added with stirring for 30 minutes, followed by centrifuging, collecting a labeled colloidal gold precipitate which is re-dissolved with the colloidal gold complex solution at 3% by volume, and stirring on a magnetic stirrer until the mixture is homogeneous, thus obtaining 3% by volume of complex solution of colloidal gold-anti-β-hCG monoclonal antibody conjugate.
(2) 3% by volume of the complex solution of colloidal gold-anti-β-hCG monoclonal antibody conjugate of the above step (1) is taken and re-dissolved with the colloidal gold complex solution at 40% by volume, then added free β antibody according to the weight ratio of anti-β-hCG monoclonal antibody to free β antibody of 1:80, wherein the free β antibody is a humanized antibody derived from hamster, followed by mixing on a magnetic stirrer, and then cast on the prepared colloidal gold adsorption pad at 50 µl/cm², followed by placing in a drying room to dry for> 4 hours, and controlling the temperature in the drying room at 18-28 °C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as the colloidal gold adsorption pad of the fifth test paper strip.

### 7. Preparation of the colloidal gold adsorption pad of the sixth test paper strip

(1) A required amount of colloidal gold is measured with a measuring cylinder, adjusting PH to 6.5-7.0 by adding 0.2 mol/L potassium carbonate solution with 0.53% by volume of the colloidal gold with stirring on a magnetic stirrer for 15 minutes, thus obtaining an adjusted colloidal gold. Anti-β-hCG monoclonal antibody is diluted with double distilled water, and labeled with the colloidal gold at 5 µg/ml, i.e. the anti-β-hCG monoclonal antibody is added into the colloidal gold, followed by stirring on a magnetic stirrer for 30 minutes, and then 0.5‰ by volume of stabilizer polyethylene glycol is added with stirring for 30 minutes, followed by centrifuging, collecting a labeled colloidal gold precipitate which is re-dissolved with the colloidal gold complex solution at 3% by volume, and stirring on a magnetic stirrer until the mixture is homogeneous, thus obtaining 3% by volume of complex solution of colloidal gold-anti-β-hCG monoclonal antibody conjugate.
(2) 3% by volume of the complex solution of colloidal gold-anti-β-hCG monoclonal antibody conjugate of the above step (1) is taken and re-dissolved with the colloidal gold complex solution at 40% by volume, then added free β antibody according to the weight ratio of anti-β-hCG monoclonal antibody to free β antibody of 1:100, wherein the free β antibody is a humanized antibody derived from hamster, followed by mixing on a magnetic stirrer, and then cast on the prepared colloidal gold adsorption pad at 50 µl/cm², followed by placing in a drying room to dry for> 4 hours, and controlling the temperature in the drying room at 18-28 °C, and the relative humidity ≤ 40%. It should be ensured that the air is unobstructed and that the air flow cannot be blown directly onto the colloidal gold adsorption pad. The dried colloidal gold adsorption pad is placed into an aluminum foil bag containing a desiccant, sealed for storage, and labeled as the colloidal gold adsorption pad of the sixth test paper strip.

Notes: The colloidal gold solution is cast, because the cast colloidal gold can be cut freely, thus it is easy to adjust the color depth of the product.

### 8. Assembly and cutting:

(1) A transparent substrate semi-finished product that has been pasted with the antibody carrying film is taken, and the colloidal gold adsorption pad of the first test paper strip is cut into 0.5cm × 30cm strip with a width of 0.5cm which is pasted on the transparent substrate close to the detection line and kept to lap about 1mm with the antibody carrying film. The water adsorption pad of the first test paper strip is compounded on the transparent substrate film close to the quality control line and lapped about 1mm with the antibody carrying film. The sample pad of the first test paper strip is compounded on the end of the colloidal gold adsorption pad of the first test paper strip away from the antibody carrying film, and lapped about 1mm therewith, labeling for use.
(2) The assembled substrate is cut into a strip test paper labeled as a first test paper strip.
(3) A transparent substrate semi-finished product that has been pasted with the antibody carrying film is taken, and the colloidal gold adsorption pad of the second test paper strip is cut into 0.5cm × 30cm strip with a width of 0.5cm which is pasted on the transparent substrate close to detection line and kept to lap about 1mm with the antibody carrying film. The water adsorption pad of the second test paper strip is compounded on the transparent substrate film close to the quality control line and lapped about 1mm with the antibody carrying film. The sample pad of the second test paper strip is compounded on the end of the colloidal gold adsorption pad of the second test paper strip away from the antibody carrying film, and lapped about 1mm therewith, labeling for use.
(4) The assembled substrate is cut into a strip test paper labeled as a second test paper strip.
(5) A transparent substrate semi-finished product that has been pasted with the antibody carrying film is taken, and the colloidal gold adsorption pad of the third test paper strip is cut into 0.5cm × 30cm strip with a width of 0.5cm which is pasted on the transparent substrate close to detection line and kept to lap about 1mm with the antibody carrying film. The water adsorption pad of the third test paper strip is compounded on the transparent substrate film close to the quality control line and lapped about 1mm with the antibody carrying film. The sample pad of the third test paper strip is compounded on the end of the colloidal gold adsorption pad of the third test paper strip away from the antibody carrying film, and lapped about 1mm therewith, labeling for use.
(6) The assembled substrate is cut into a strip test paper labeled as a third test paper strip.
(7) A transparent substrate semi-finished product that has been pasted with the antibody carrying film is taken, and the colloidal gold adsorption pad of the fourth test paper strip is cut into 0.5cm × 30cm strip with a width of 0.5cm which is pasted on the transparent substrate close to detection line and kept to lap about 1mm with the antibody carrying film. The water adsorption pad of the fourth test paper strip is compounded on the transparent substrate film close to the quality control line and lapped about 1mm with the antibody carrying film. The sample pad of the fourth test paper strip is compounded on the end of the colloidal gold adsorption pad of the fourth test paper strip away from the antibody carrying film, and lapped about 1mm therewith, labeling for use.
(8) The assembled substrate is cut into a strip test paper labeled as a fourth test paper strip.
(9) A transparent substrate semi-finished product that has been pasted with the antibody carrying film is taken, and the colloidal gold adsorption pad of the fifth test paper strip is cut into 0.5cm × 30cm strip with a width of 0.5cm which is pasted on the transparent substrate close to detection line and kept to lap about 1mm with the antibody carrying film. The water adsorption pad of the fifth test paper strip is compounded on the the transparent substrate film close to the quality control line and lapped about 1mm with the antibody carrying film. The sample pad of the fifth test paper strip is compounded on the end of the colloidal gold adsorption pad of the fifth test paper strip away from the antibody carrying film, and lapped about 1mm therewith, labeling for use.
(10) The assembled substrate is cut into a strip test paper labeled as a fifth test paper strip.
(11) A transparent substrate semi-finished product that has been pasted with the antibody carrying film is taken, and the colloidal gold adsorption pad of the sixth test paper strip is cut into 0.5cm × 30cm strip with a width of 0.5cm which is pasted on the transparent substrate close to detection line and kept to lap about 1mm with the antibody carrying film. The water adsorption pad of the sixth test paper strip is compounded on the transparent substrate film close to the quality control line and lapped about 1mm with the antibody carrying film. The sample pad of the sixth test paper strip is compounded on the end of the colloidal gold adsorption pad of the sixth test paper strip away from the antibody carrying film, and lapped about 1mm therewith, labeling for use.
(12) The assembled substrate is cut into a strip test paper labeled as a sixth test paper strip.

### Example 2

The present example provides a test strip for semi-quantitative detection of human chorionic gonadotropin, which is different from the test strip of example 1 in that free β antibody is an equine antibody, and the interval between the detection line 6 and the quality control line 7 is 0.3cm.

The present example also provides a method for preparing the above test strip for semi-quantitative detection of human chorionic gonadotropin, which is different from the preparation method of example 1 in that the free β antibody used is an equine antibody; during the preparation process of the antibody carrying film, the concentration of anti-α-HCG monoclonal antibody is 2 mg/ml, the concentration of anti-mouse IgG polyclonal antibody is 1 mg/ml, and the interval between the detection line and the quality control line is 0.3cm; and the sealing treatment solution used includes 0.08Mol buffer solution, 0.3wt% sugar, 0.8 wt% sealing protein, and 0.03 wt% preservative, wherein the buffer solution is phosphate buffer, the sugar is trehalose, the preservative is NaN₃, and the sealing protein is casein.

### Example 3

The present example provides a test strip for semi-quantitative detection of human chorionic gonadotropin, which is different from the test strip of example 1 in that free β antibody is a rabbit antibody, and the interval between the detection line 6 and the quality control line 7 is 1.0cm.

The present example also provides a method for preparing the above test strip for semi-quantitative detection of human chorionic gonadotropin, which is different from the preparation method of example 1 in that the free β antibody used is a rabbit antibody; during the preparation process of the antibody carrying film, the concentration of anti-α-HCG monoclonal antibody is 3 mg/ml, the concentration of anti-mouse IgG polyclonal antibody is 2 mg/ml, and the interval between the detection line and the quality control line is 0.7cm; and the sealing treatment solution used includes 0.12Mol buffer solution, 0.7wt% sugar, 1.2 wt% sealing protein, and 0.07 wt% preservative, wherein the buffer solution is phosphate buffer, the sugar is sucrose, the preservative is thimerosal, and the sealing protein is bovine serum protein.

### Example 4

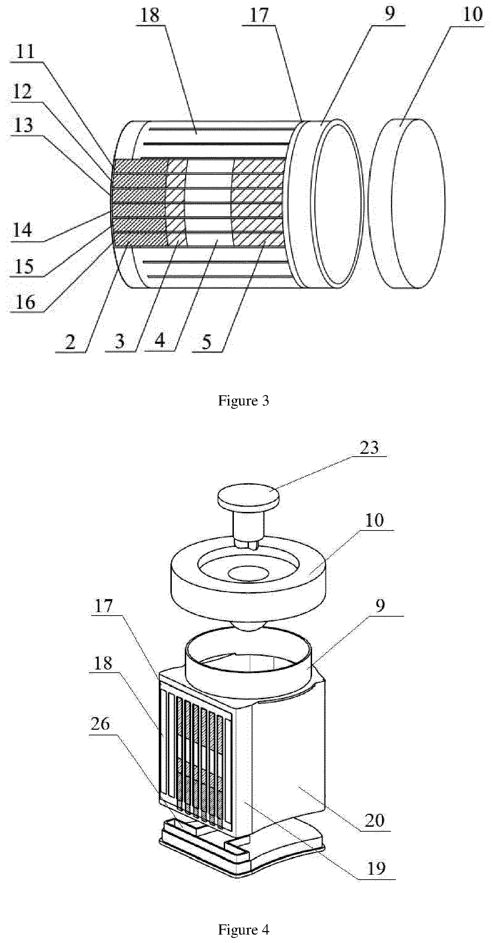

As shown in Figure 3, the present example provides a reagent cup for semi-quantitative detection of human chorionic gonadotropin, which comprises the test paper for semi-quantitative detection of human chorionic gonadotropin prepared in example 1, and further comprises a cup bod 9, a cup cover 10 and a cartridge 17. The cartridge 17 is placed vertically inside the cup body 9, and the cup cover 10 is detachably closed on the cup body 9. As shown in Figure 2, the cartridge 17 is provided with six or more parallel slots 18, and the slots 18 have an opening near the bottom of the cup body 9. The first test paper strip 11, the second test paper strip 12, the third test paper strip 13, the fourth test paper strip 14, the fifth test paper strip 15 and the sixth test paper strip 16 are successively inserted into the six slot 18 via the opening. The sample pad 2 of the test paper strip extends from the opening to the outside of the cartridge 17. Cartridge 17 is made of plastic, of course, as an alternative embodiment, cartridge 17 can also be made of other transparent materials.

Specifically, the structure of each of the first test paper strip 11, the second test paper strip 12, the third test paper strip 13, the fourth test paper strip 14, the fifth test paper strip 15 and the sixth test paper strip 16 is shown in Figure 1, which respectively includes a substrate 1 and a sample pad 2, a colloidal gold adsorption pad 3, an antibody carrying film 4 and a water adsorption pad 5 sequentially adhered to the substrate. The sample pad 2, the colloidal gold adsorption pad 3, the antibody carrying film 4 and the water adsorption pad 5 are partially overlapped and lapped with each other. Specifically, the antibody carrying film 4 is located under the colloidal gold adsorption pad 3 and the water adsorption pad 5, the sample pad 2 is located above the colloidal gold adsorption pad 3, and the length of the overlapping part is 1mm.

The antibody carrying film 4 is provided with a detection line 6 on an end close to the colloidal gold adsorption pad 3 and a quality control line 7 on an end close to the water adsorption pad 5. The detection line 6 is coated with an anti-α-hCG monoclonal antibody. The quality control line 7 is coated with an anti-mouse IgG polyclonal antibody.

The colloidal gold adsorption pad 3 of the first test paper strip 11 is adsorbed with colloidal gold-anti-β-hCG monoclonal antibody conjugate. The colloidal gold adsorption pad 3 of the second test paper strip 12, the third test paper strip 13, the fourth test paper strip 14, the fifth test paper strip 15 and the sixth test paper strip 16 is adsorbed with colloidal gold-anti-β-hCG monoclonal antibody conjugate and free β antibody. The free β antibody is non-murine antibody. In the example, the free β antibody is a humanized antibody derived from hamster.

Moreover, the detection line 6 and the quality control line 7 are arranged in parallel with a pitch of 0.3-1.0 cm. In the example, the pitch is 0.5 cm. Moreover, a protective film 8 is disposed on the sample pad 2 and the water adsorption pad 3 respectively. Moreover, the antibody carrying film 4 is a nitrocellulose film with a pore size of 3-10 µm

When in use, the cup cover 10 is opened, the urine sample is collected in the cup body 9, the cup cover 10 is fitted on the cup body 9, the cup body 9 is placed upright, and the sample pads 2 of the six test paper strips are immersed in the sample. The sample is inhaled into the test paper strip from the sample pad 2 due to the capillarity, and then passes through the colloidal gold adsorption pad 3 and the antibody carrying film 4 successively to reach to the water adsorption pad 5, the hCG in the sample binds with the colloidal gold-anti-β-hCG monoclonal antibody conjugate or free β antibody adsorbed in the colloidal gold adsorption pad 3. With the flow of liquid, the hCG, bound to the colloidal gold-anti-β-hCG monoclonal antibody conjugate, reaches to the antibody carrying film 4, and binds to anti-α-hCG monoclonal antibody coated in the detection line and anti-mouse IgG polyclonal antibody coated in the quality control line, respectively, and develops color at the detection line and the quality control line because it has colloidal gold labeling. While the hCG, bound to free β antibody, does not develop color at the detection line because it does not have colloidal gold labeling, and does not bind to anti-mouse IgG polyclonal antibody coated in the quality control line because the free β antibody is not murine antibody. Even if the concentration of hCG in the sample is high and a large amount of hCG binds to free β antibody, it will not cause most of antibodies in the quality control line to bind to hCG-free β antibody conjugate, so that the quality control line will not develop color. As a result, the samples with higher hCG concentration can be measured, and the detectable quantity can reach to 10000mIU/ml, making the range of concentration detection wider. Since the weight ratios of colloidal gold-anti-β-hCG monoclonal antibody conjugate to free β antibody in the colloidal gold adsorption pad of six test paper strips are different, the detection line and quality control line of the six test paper strips will show different color development. According to the color development, the concentration of hCG in the sample can be detected semi-quantitatively.

### Example 5

As shown in Figures 4-6, the present example provides a reagent cup for semi-quantitative detection of human chorionic gonadotropin, which comprises the test paper for semi-quantitative detection of human chorionic gonadotropin prepared in example 1, and further comprises a cup bod 9 and a cartridge 17.

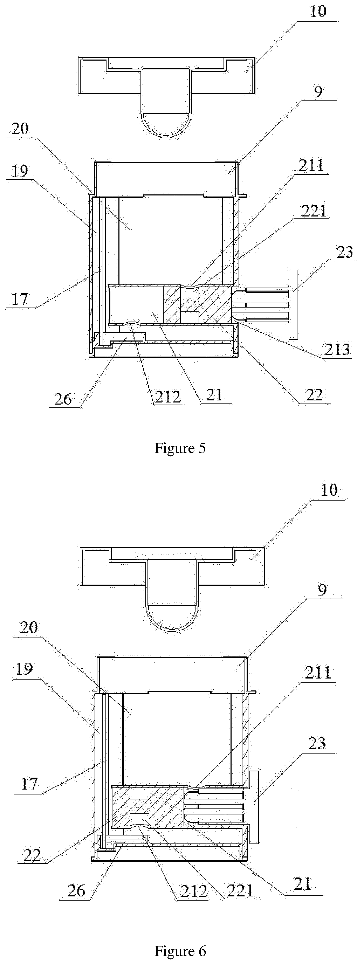

The cup body 9 is provided with a detection cavity 19, a liquid storage cavity 20 and a liquid guide cavity 21. The liquid guide cavity 21 is provided with a first through hole 211 intercommunicating with the liquid storage cavity at an upper part thereof and a second through hole 212 intercommunicating with the detection cavity 19 at a lower part thereof. A piston 22 sliding along the liquid guide cavity is disposed inside the liquid guide cavity 21, has an initial state for intercommunicating the liquid guide cavity 21 with the liquid storage cavity 20 and a detection state for intercommunicating the liquid chamber guide cavity 21 with the detection chamber 19. A third through hole 213 is disposed on a cup wall of the cup body 9 away from the detection chamber 19 and intercommunicated with the liquid guide cavity 21. A liquid storage tank 221 is disposed on the piston 22. As shown in Figure 5, when the piston 22 is in the initial state, the liquid storage tank 221 of the piston is aligned with the first through hole 211, so that the liquid guide cavity 21 is intercommunicated with the liquid storage cavity 20; when the piston 22 is in the detection state, the liquid storage tank 221 of the piston is aligned with the second through hole 212, so that the liquid guide cavity 21 is intercommunicated with the detection cavity 19.

Moreover, a liquid guide tank 26 is provided inside the bottom of the cup body 9, the liquid guide tank 26 intercommunicates with the detection cavity 19, and the liquid guide tank 26 is located below the second through hole 212, so that the liquid guide tank 26 intercommunicates with the second through hole 212.

The structure and number of the cartridge 17 and the test paper strip are the same as those in Example 4, except that the cartridge 17 inserted with the test paper strip is vertically placed inside the detection cavity 19.

By setting the cartridge 17, the reagent cup can fix the test paper strip in the detection cavity 19 of the reagent cup. When in use, the cup body can be kept upright, so that the test paper strip can also be kept upright, which is convenient for sample absorption. The reagent cup is configured to comprise six test paper strips, and the hCG concentration in urine can be semi-quantitatively detected according to the color development of different test paper strips. The detectable concentration range is wider, and there is no need to identify by comparing the degree of color development, thus the test result is more accurate. The detection cavity 19 comprising the cartridge and the test paper, and a liquid storage cavity 20 for collecting urine, are configured to make the test paper in the detection cavity 19 separate from the urine sample under the initial state. The piston 22 and the liquid storage tank 221 disposed on the piston are configured to make the urine enter into the liquid storage tank 221 from the liquid storage cavity 20 via the first through hole 211. When the piston 22 slides in the liquid guide cavity 21 to the detection state, the urine enters into the liquid guide tank 26 from the liquid storage tank 221 via the second through hole 212, thus the test paper strip in the cartridge 17 of the detection cavity 19 contacts with the urine sample, so as to complete the detection. Through the above structure, the time when starts the test can be more conveniently controlled, and the test can be started after the sample collection is completed, so as to prevent the problem that the test result is not accurate enough when collecting samples while simultaneously performing the test, making the result more accurate and easy to operate. The liquid guide cavity 21 intercommunicates with the third through hole 213, so that the sliding of the piston 22 can be easily controlled. The liquid storage tank 221 is configured to make the amount of urine samples introduced into the detection cavity 19 constant, thereby preventing excessive urine samples from entering the detection cavity 19 and affecting the test results. The accuracy of the test results is ensured. When using the reagent cup, the user only needs to collect the urine sample directly in the cup body, and then pushes the piston 22, the test and result reading can be performed, which is convenient and hygienic.

The reagent cup further includes a cup cover 10 detachably closed on the cup body 9. A boost member 23 for boosting the piston 22 is provided on the cup cover 10, and detachably fitted on the cup cover 10. The boost member 23 is configured to more easily push the piston 22 from the initial state to the detection state. When the reagent cup is not used, the boost member 23 can be fitted on the cup cover 10 to facilitate storage and prevent its loss.

When in use, the cup cover 10 is opened, the urine sample is collected in the liquid storage cavity 20 of the cup body 9, the cup cover 10 is fitted on the cup body 9, the cup body 9 is placed upright. At this time, as shown in Figure 5, when the reagent cup is in the initial state, the urine sample in liquid storage cavity 20 enters into the liquid storage tank 221 of the piston 22 through the first through hole 211. The boost member 23 fitted on the cup cover 10 is taken down, and inserted into the third through hole 213, and the piston 22 is pushed to the detection state through the boost member 23. As shown in Figure 6, the urine sample in the liquid storage tank 221 flows into the liquid guide tank 26 at the bottom through the second through hole 212, and then flows into the detection cavity 19, the sample pads 2 of six test paper strips in detection cavity 19 are immersed in the sample. The sample is inhaled into the test paper strip from the sample pad 2 due to the capillarity, and then passes through the colloidal gold adsorption pad 3 and the antibody carrying film 4 successively to reach to the water adsorption pad 5, the hCG in the sample binds with the colloidal gold-anti-β-hCG monoclonal antibody conjugate or free β antibody adsorbed in the colloidal gold adsorption pad 3. With the flow of liquid, the hCG, bound to the colloidal gold-anti-β-hCG monoclonal antibody conjugate, reaches to the antibody carrying film 4, and binds to anti-α-hCG monoclonal antibody coated in the detection line and anti-mouse IgG polyclonal antibody coated in the quality control line, respectively, and develops color at the detection line 6 and the quality control line 7 because it has colloidal gold labeling. While the hCG, bound to free β antibody, does not develop color at the detection line 6 because it does not have colloidal gold labeling, and does not bind to anti-mouse IgG polyclonal antibody coated in the quality control line 7 because the free β antibody is not murine antibody. Since the weight ratios of colloidal gold-anti-β-hCG monoclonal antibody conjugate to free β antibody in the colloidal gold adsorption pad of six test paper strips are different, the detection line 6 of the six test paper strips will show different color development. According to the color development, the concentration of hCG in the sample can be detected semi-quantitatively. Compared with two test paper strips, the six test paper strips of the present application have a wider detectable concentration range, and there is no need to identify by comparing the degree of color development, thus the test result is more accurate. When using the reagent cup, user only needs to collect the urine sample directly in the cup body, and then pushes the piston 22, the test and result reading can be performed, which is convenient and hygienic.

### Example 6

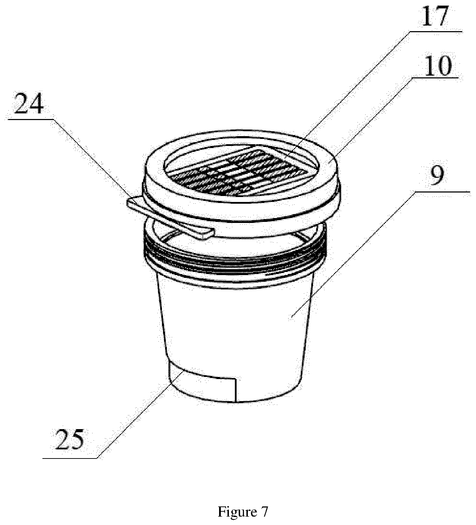

As shown in Figure 7, the present example provides a reagent cup for semi-quantitative detection of human chorionic gonadotropin, which comprises a cup bod 9, a cartridge 17 and the test paper of example 1, and further comprises a cup cover 10, which is detachably fitted on the cup body 9. The structure and number of the cartridge 17 and the test paper strip are the same as those in Example 4, except that the cartridge 17 is fixed in parallel inside the cup cover 10.

By setting the cartridge 17, the reagent cup can fix the test paper strip in the detection cavity 19 of the reagent cup. When in use, the cup body 9 is in an on its side state, which is convenient for absorbing sample with the test paper strip. The reagent cup is configured to comprise six test paper strips, and the hCG concentration in urine can be semi-quantitatively detected according to the color development of different test paper strips. The detectable concentration range is wider, and there is no need to identify by comparing the degree of color development, thus the test result is more accurate. By fixing the cartridge 17 on the cup cover 10, the test paper strip in the cartridge 17 can be separated from the urine sample in the cup body10 when the test is not started. At the beginning of the test, the test can be started by tightening the cup cover 10, and placing the reagent cup on its side, so as to prevent the problem that the test result is not accurate enough when collecting samples while simultaneously performing the test, make the result more accurate and easy to operate.

A support body 24 is disposed on an outer edge of the cup cover 20 for fixing the reagent cup placed on its side, and a scale line 25 is arranged on the cup body 10. The support body 24 is configured to dispose on the edge of the cup cover 10, the reagent cup is fixed when it is placed on its side, preventing the reagent cup from rolling. When collecting urine, it is necessary to ensure that the urine exceeds the scale line 25, ensuring that the urine samples are sufficient when the reagent cup is placed on its side, so that the test paper strip can carry out adsorption detection successfully.

When in use, the cup cover10 is opened, the urine sample is collected in the cup body 9 to make the volume of urine sample exceed the scale line25 on the cup body 9. The cup cover 10 is fitted on the cup body 9 and tightened, the reagent cup is placed on its side and fixed with the support body 24. The sample pads 2 of six test paper strips are immersed in the sample. The sample is inhaled into the test paper strip from the sample pad 2 due to the capillarity, and then passes through the colloidal gold adsorption pad 3 and the antibody carrying film 4 successively to reach to the water adsorption pad 5, the hCG in the sample binds with the colloidal gold-anti-β-hCG monoclonal antibody conjugate or free β antibody adsorbed in the colloidal gold adsorption pad 3. With the flow of liquid, the hCG, bound to the colloidal gold-anti-β-hCG monoclonal antibody conjugate, reaches to the antibody carrying film 4, and binds to anti-α-hCG monoclonal antibody coated in the detection line and anti-mouse IgG polyclonal antibody coated in the quality control line, respectively, and develops color at the detection line 6 and the quality control line 7 because it has colloidal gold labeling. While the hCG, bound to free β antibody, does not develop color at the detection line 6 because it does not have colloidal gold labeling, and does not bind to anti-mouse IgG polyclonal antibody coated in the quality control line 7 because the free β antibody is not murine antibody. Since the weight ratios of colloidal gold-anti-β-hCG monoclonal antibody conjugate to free β antibody in the colloidal gold adsorption pad of six test paper strips are different, the detection line 6 of the six test paper strips will show different color development. According to the color development, the concentration of hCG in the sample can be detected semi-quantitatively. Compared with two test paper strips, the six test paper strips of the present application have a wider detectable concentration range, and there is no need to identify by comparing the degree of color development, thus the test result is more accurate. When using the reagent cup, user only needs to collect the urine sample directly in the cup body, and then pushes the piston 22, the test and result reading can be performed, which is convenient and hygienic.

### Example 7

The present example provides a method for using the reagent cup for semi-quantitative detection of human chorionic gonadotropin in Example 4, which includes steps of: equilibrating the sample and the reagent cup at room temperature; then opening the cup cover 10 and collecting the urine sample in the cup body 9, fitting the cup cover 10 on the cup body 9, placing the cup body 9 upright down, immersing the sample pad 2 of the six test paper strips in the sample, and observing whether the detection line and quality control line of the six test paper strips develop color within 5 minutes. The test results include the following situations:

**Table 1: Interpretation of displayed results of test paper strips**

| Displayed Results | hCG Concentration Range |
|---|---|
| The quality control lines of the six test paper strips do not develop prunosus | Invalid |
| The quality control lines of the six test paper strips develop prunosus; | Negative (-): Less than 5mIU/ml |
| The detection lines of the six test paper strips do not develop prunosus | |
| The quality control lines of the six test paper strips develop prunosus; | Positive (+): 5-25mIU/ml |
| The detection line of the first test paper strip develops prunosus; | |
| The detection lines of the rest test paper strips do not develop prunosus | |
| The quality control lines of the six test paper strips develop prunosus; | Positive (++): 25-100mIU/ml |
| The detection lines of the first and second test paper strips develop prunosus; | |
| The detection lines of the rest test paper strips do not develop prunosus | |
| The quality control lines of the six test paper strips develop prunosus; | Positive (+++): 100-500mIU/ml |
| The detection lines of the first, second and third test paper strips develop prunosus; | |
| The detection lines of the rest test paper strips do not develop prunosus | |
| The quality control lines of the six test paper strips develop prunosus; | Positive (++++): 500-2500mIU/ml |
| The detection lines of the first, second, third and fourth test paper strips develop prunosus; | |
| The detection lines of the rest test paper strips do not develop prunosus | |
| The quality control lines of the six test paper strips develop prunosus; | Positive (+++++): 2500-10000mIU/ml |
| The detection lines of the first, second, third, fourth and fifth test paper strips develop prunosus; | |
| The detection line of the sixth test paper strip does not develop prunosus | |
| The quality control lines of the six test paper strips develop prunosus; | Positive (++++++): Greater than or equal to 10000 mIU/ml |
| The detection lines of the six test paper strips develop prunosus | |

The test begins on the day when menstruation should come but does not come. If the test begins in advance, it should be performed again on the expected date of menstruation. If menstruation is delayed, the test should be performed again after three days. If the menstrual period is irregular, the longest menstrual period in the most recent months should be calculated before the test is performed. If you don't know when menstruation is expected to begin, you should perform the test at least nineteen days after the last time you had sex without protective measures.

### Example 8

The present example provides a use of the test paper for semi-quantitative detection of human chorionic gonadotropin in Example 4 for detecting human pregnancy cycle, ectopic pregnancy, miscarriage and trophoblastic disease.

The urine sample is tested using the method of using the reagent cup in Example 7, and the concentration range of human chorionic gonadotropin in the urine sample is semi-quantitatively detected according to the Interpretation in Table 1.

Human pregnancy cycle is identified according to reference values of hCG of pregnant women described in "*Chinese Clinical Test Operating Procedures*". The reference value of hCG is 5-50 IU/ml for 0.2-1 week of pregnancy, the reference value of hCG is 50-500 IU/ml for 1-2 weeks of pregnancy, the reference value of hCG is 100-5000 IU/ml for 2-3 weeks of pregnancy, the reference value of hCG is 500-10000 IU/ml for 3-4 weeks of pregnancy, the reference value of hCG is 1000-50,000 IU/ml for 4-5 weeks of pregnancy, the reference value of hCG is 10,000-100,000 IU/ml for 5-6 weeks of pregnancy, the reference value of hCG is 15,000-200,000 IU/m for 6-8 weeks of pregnancy, the reference value of hCG is 10,000-100,000 IU/ml for 8-12 weeks of pregnancy.

In the aspect of clinical diagnosis, the concentration range of human chorionic gonadotropin in the urine samples is detected semi-quantitatively to diagnose ectopic pregnancy, miscarriage and trophoblastic disease, and observe the course of disease. Specifically, in the diagnosis of ectopic pregnancy, the hCG of ectopic pregnancy is often 312-625mIU/ml, and hCG result can still be positive after three days of uterine bleeding, so hCG test can be used as a method to distinguish ectopic pregnancy from other acute abdomen. In the diagnosis of miscarriage, if there is still placental tissue remaining in the uterus, hCG result can still be positive, while when complete abortion or stillbirth occurs, hCG result changes from positive to negative. Therefore, hCG test can be used as a reference basis for fetal protection or uterine aspiration treatment. In the diagnosis of threatened abortion, if the urine hCG has been kept at a high level, there will be no inevitable abortion, but if hCG is below 2500mIU/ml and gradually decreases, there is the possibility of abortion or stillbirth, and when hCG falls to 600mIU/ml, there will be inevitable abortion. Therefore, in the treatment of protecting fetus, if hCG continues to decline, it means that the protecting fetus is ineffective, while if hCG continues to rise, it means that the protecting fetus is successful. In addition, urinary hCG levels in patients with hydatidiform mole, malignant hydatidiform mole and chorionic epithelioma are significantly increased, ranges from 100,000 to millions of mIU/ml. The urine hCG of the patients with trophoblastic tumor should be less than 50mIU/ml at 3 weeks and negative at 8-12 weeks after operation. If the hCG does not decrease or change to negative, it may indicate that there may be residual lesions.

### Comparative Example 1

This comparative example provides a test paper for semi-quantitative quantitative detection of human chorionic gonadotropin similar to that in Example 1, except that the free β antibody used is a murine antibody, and a national level human chorionic gonadotropin standard is prepared into a standard solution with a concentration of 10000mIU/ml. When test is performed using the test paper, the result is that of the six test paper strips, the quality control lines of the fourth, fifth, and sixth test paper strips do not develop color, thus the result is invalid.

### Comparative Example 2

The national level human chorionic gonadotropin standard is prepared into a standard solution with a concentration of 5mIU/ml. When test is performed using hCG cycle test paper disclosed in Chinese Patent CN108761099A and the hCG cycle test kit including the test paper, the result is that of the quality control lines of the two test paper strips develop color, and the detection lines of the two test paper strips do not develop color.

### Experimental example 1

1. The required reagents are as follows:
   0 mIU/ml sample solution: protein-containing phosphate buffer (PBS) is 0 mIU/ml of the HCG sample solution.
   5m IU/ml, 25 mIU/ml, 100mIU/ml, 500mIU/ml, 2500mIU/ml, and 10000mIU/ml human chorionic gonadotropin (hCG) sample solution prepared by human chorionic gonadotropin (hCG) standard and the protein-containing phosphate buffer (0.01M PBS) to reach a corresponding concentration respectively.
   500m IU/ml human luteinizing hormone (hLH): After hLH standard is re-dissolved with the protein-containing phosphate buffer (PBS), 0m IU/ml HCG sample solution is used to prepare hLH A solution with a concentration of 500m IU/ml; 5m IU/ml human chorionic gonadotropin sample solution is used to prepare hLH B solution with a concentration of 500m IU/ml.
   1000m IU/ml human follicle stimulating hormone (hFSH): After the hFSH standard is re-dissolved with the protein-containing phosphate buffer (PBS), 0m IU/ml HCG sample solution is used to prepare hFSH A solution with a concentration of 1000m IU/ml; 5m IU/ml human chorionic gonadotropin sample solution is used to prepared hFSH B solution with a concentration of 1000m IU/ml.
   1000µ IU/ml human thyroid stimulating hormone (hTSH): After the hTSH standard is re-dissolved with the protein-containing phosphate buffer (PBS), 0m IU/ml HCG sample solution is used to prepare hTSH A solution with a concentration of 1000µ IU/ml; 5m IU/ml human chorionic gonadotropin sample solution is used to prepare hTSH B solution with a concentration of 1000µ IU/ml; and 5m IU/ml human chorionic gonadotropin sample solution is used to prepare hTSH B solution with a concentration of 1000µ IU/ml .
2. Testing Physical properties:
   Testing appearance properties: The test paper strip is taken out and observed with eyesight. The paper strip should be neat and complete, have no burrs, no damage, and no pollution, and be firmly attached with the material.

Film strip width: Two of each six kinds of sensitivity test paper strips are taken and measured the width of the film on the paper strips with vernier caliper, and the average value of the two measurement results is calculated.

Chromatographic status: regular, the colloid gold not layered with water, and a background is basically clear within 3-5 minutes.

Liquid migration speed: two test paper strips, of each of the six sensitivities test paper strips are taken, and the operation is carried out according to the method used in Example 7. The time used is recorded as (t) from the time when the test paper strip is immersed in the sample solution with a stopwatch (with an accuracy of 0.01s) until the liquid reaches the boundary between the antibody carrying film and the water adsorption pad. The length is recorded as (L) from the sample loading end of the sample pad to the boundary between the antibody carrying film and the water adsorption pad measured with Vernier caliper (precision 0.02mm). The migration velocity, i.e. L/t is calculated. The average values of repeated measurement of two test paper strips, of each of six sensitivities test paper strips, are taken respectively. The results are consistent with the liquid migration speed of no less than 10mm/min. The chromatographic state is regular, the colloidal gold is not delaminated with water, and the background is basically clear within 5 minutes.

### 3. The minimum detectable quantity of the test:

A national level human chorionic gonadotropin standard is prepared into standard solutions with concentrations of 2.5 mIU/ml, 5 mIU/ml, and 10 mIU/ml, respectively. The standard solution with each concentration is tested three times, using the reagent cup of Example 4 of the present application, and the results are observed within 5 minutes, showing that the minimum detectable quantity of the first test paper strip is 5 mIU/ml. A national level human chorionic gonadotropin standard is prepared into standard solutions with concentrations of 12.5 mIU/ml, 25 mIU/ml, and 50 mIU/ml. The standard solution with each concentration is tested three times, and the results are observed within 5 minutes, showing that the minimum detectable quantity of the second test paper strip is 25 mIU/ml. A national level human chorionic gonadotropin standard is prepared into standard solutions with concentrations of 50 mIU/ml, 100 mIU/ml, and 200 mIU/ml. The standard solution with each concentration is tested three times, and the results are observed within 5 minutes, showing that the minimum detectable quantity of the third test paper strip is 100 mIU/ml. A national level human chorionic gonadotropin standard is prepared into standard solutions with concentrations of 400mIU/ml, 500mIU/ml, and 600mIU/ml. The standard solution with each concentration is tested three times, and the results are observed within 5 minutes, showing that the minimum detectable quantity of the fourth test paper strip is 500 mIU/ml. A national level human chorionic gonadotropin standard is prepared into standard solutions with concentrations of 2400mIU/ml, 2500mIU/ml, and 2600mIU/ml. The standard solution with each concentration is tested three times, and the results are observed within 5 minutes, showing that the minimum detectable quantity of the fifth test paper strip is 2500 mIU/ml. A national level human chorionic gonadotropin standard is prepared into standard solutions with concentrations of 9900mIU/ml, 10000mIU/ml, and 10100mIU/ml. The standard solution with each concentration is tested three times, and the results are observed within 5 minutes, showing that the minimum detectable quantity of the sixth test paper strip is 10000 mIU/ml.

### 5. Accuracy and repeatability of test:

national level human chorionic gonadotropin standard is prepared into standard solutions with concentrations of 5mIU/m, 25mIU/ml, 100mIU/ml, 500mIU/ml, 2500mIU/ml, and 10000mIU/ml. The standard solution with each concentration is tested twenty times to obtain the test result. The forward difference between the test results and the labeled value of the corresponding standard solution does not exceed one order of magnitude, There is no backward difference. The positive standard solution does not show a negative result.

### 5. Specificity:

Negative specificity: 500m IU/ml hLH A solution, 1000m IU/ml hFSH A solution, 1000µ IU/ml hTSH A solution are tested for 3 times respectively. Results of the three times of each solution show that the detection lines are negative.

Positive specificity: 500m IU/ml hLH B solution, 1000m IU/ml hFSH B solution, and 1000µ IU/ml hTSH B solution are tested for 3 times respectively. Results of the three times of each solution show that the detection line is positive.

### 6. Stability of test:

Physical properties, accuracy, repeatability, and specificity of the test paper strip and the reagent cup are tested after one month after the expiration date, the results meet the requirements.

It is apparent that the above embodiments are merely examples for clarity of illustration, and are not intended to limit the embodiments. Other variations or modifications in various forms may be made by those skilled in the art in view of the above description. There is no need and no way to present all of embodiments herein. The obvious variations or modifications derived therefrom are still within the scope of protection of the present application.

## Claims

1. A test paper for semi-quantitative detection of human chorionic gonadotropin, **characterized by** comprising at least three test paper strips, each including
a substrate (1), and
a sample pad (2), a colloidal gold adsorption pad (3), an antibody carrying film (4) and a water absorption pad (5) sequentially adhered to the substrate (1);
wherein the sample pad (2), the colloidal gold adsorption pad (3), the antibody carrying film (4), and the water absorption pad (5) are partially overlapped and lapped with each other;
the antibody carrying film (4) is provided with a detection line (6) on an end close to the colloidal gold adsorption pad (3) and a quality control line (7) on an end close to the water adsorption pad (5); the detection line (6) is coated with an anti-human chorionic gonadotropin α-hCG monoclonal antibody; the quality control line (7) is coated with an anti-mouse IgG polyclonal antibody;
the colloidal gold adsorption pad (3) of one of the at least three test paper strips is adsorbed with colloidal gold-anti-human chorionic gonadotropin β-hCG monoclonal antibody conjugate, and the colloidal gold adsorption pad (3) of the rest of the at least three test paper strips is adsorbed with colloidal gold-anti-human chorionic gonadotropin β-hCG monoclonal antibody conjugate and a free β antibody with different weight ratio; wherein the free β antibody is a non-murine antibody.

2. The test paper for semi-quantitative detection of human chorionic gonadotropin according to claim 1, **characterized in that**, the free β antibody is a humanized, equine, rabbit or goat antibody.

3. The test paper for semi-quantitative detection of human chorionic gonadotropin according to claim 1 or 2, **characterized in that**, the test paper comprises six test paper strips of a first test paper strip (11), a second test paper strip (12), a third test paper strip (13), a fourth test paper strip (14), a fifth test paper strip (15) and a sixth test paper strip (16).

4. The test paper for semi-quantitative detection of human chorionic gonadotropin according to claim 3, **characterized in that**, the weight ratios of colloidal gold-anti-human chorionic gonadotropin β-hCG monoclonal antibody conjugate to free β antibody adsorbed on colloidal gold adsorption pad of the second test paper strip (12), the third test paper strip (13), the fourth test paper strip (14), the fifth test paper strip (15), and the sixth test paper strip (16) are 1:1, 1:2, 1:40, 1:80, 1:100, respectively.

5. A method for preparing a test paper for semi-quantitative detection of human chorionic gonadotropin according to any one of claims 1-4, **characterized by** comprising the steps of:
S1: coating an antibody carrying film with anti-human chorionic gonadotropin α-hCG monoclonal antibody and anti-mouse IgG polyclonal antibody, respectively, to obtain a detection line and a quality control line, performing sealing treatment in a sealing treatment solution, and drying for use;
S2: taking colloidal gold and adjusting pH thereof, adding anti-human chorionic gonadotropin β-hCG monoclonal antibody therein, adding a stabilizer with stirring, centrifuging and collecting a precipitate, redissolving the precipitate with a colloidal gold complex solution to obtain colloidal gold-anti-human chorionic gonadotropin β-hCG monoclonal antibody conjugate complex solution;
S3: casting the colloidal gold-anti-human chorionic gonadotropin β-hCG monoclonal antibody conjugate complex solution obtained in step S2 on a colloidal gold adsorption pad of one test paper strip, followed by drying, sealing and storing for use;
S4: adding free β antibody to the colloidal gold-anti-human chorionic gonadotropin β-hCG monoclonal antibody conjugate complex solution obtained in step S2, and mixing well to obtain different mixed complex solutions with different weight ratio of colloidal gold-anti-human chorionic gonadotropin β-hCG monoclonal antibody conjugate to free β antibody, casting the different mixed complex solutions on colloidal gold adsorption pads of the rest of test paper strips, followed by drying, sealing and storing for use;
S5: taking a sample pad, a colloidal gold adsorption pad, an antibody carrying film, and a water adsorption pad which are pasted sequentially along a length direction of a substrate in a partially overlapping manner, and obtaining a test paper strip, and preparing at least three test paper strips in this way;
wherein the free β antibody is a non-murine antibody.

6. The method according to claim 5, **characterized in that**, there are six test paper strips of a first test paper strip, a second test paper strip, a third test paper strip, a fourth test paper strip, a fifth test paper strip and a sixth test paper strip.

7. The method according to claim 6, **characterized in that**, in the first, second, and third test paper strips, the anti-human chorionic gonadotropin β -hCG monoclonal antibody is labeled with colloidal gold at 4 µg/ml, and in the fourth, fifth, and sixth test paper strips, the anti-human chorionic gonadotropin β-hCG monoclonal antibody is labeled with colloidal gold at 5 µg/ml.

8. The method according to claim 6 or 7, **characterized in that**, in the second, third, fourth, fifth, and sixth test paper strips, the weight ratios of the anti-human chorionic gonadotropin β-hCG monoclonal antibody to the free β antibody are 1:1, 1:2, 1:40, 1:80, and 1:100, respectively.

9. The method according to any one of claims 5-8, **characterized in that**, the anti-human chorionic gonadotropin α-hCG monoclonal antibody has a concentration of 2-3 mg/ml, the anti-mouse IgG polyclonal antibody has a concentration of 1-2 mg/ml.

10. A reagent cup for semi-quantitative detection of human chorionic gonadotropin, **characterized by** comprising the test paper for semi-quantitative detection of human chorionic gonadotropin according to any one of claims 1-4 or the test paper for semi-quantitative detection of human chorionic gonadotropin prepared by the method according to any one of claims 5-9.

11. The reagent cup for semi-quantitative detection of human chorionic gonadotropin according to claim 10, **characterized by** further comprising
a cup body (9), and
a cartridge (17) disposed inside the reagent cup and provided with parallel slots (18), wherein, one end of the slot (18) is provided with an opening for the test paper strip to be inserted therein, a sample pad (2) of a test paper strip extends from the opening to an outside of the cartridge (17).

12. The reagent cup for semi-quantitative detection of human chorionic gonadotropin according to claim 11, **characterized by** further comprising a cup cover (10) detachably closed on the cup body (9).

13. The reagent cup for semi-quantitative detection of human chorionic gonadotropin according to claim 11 or 12, **characterized in that**, the cartridge (17) is disposed vertically inside the cup body (9), and the opening of the slot (18) is close to the bottom of the cup body (9).

14. The reagent cup for semi-quantitative detection of human chorionic gonadotropin according to claim 13, **characterized in that**, the cup body (9) is provided with a detection cavity (19), a liquid storage cavity (20) and a liquid guide cavity (21);
wherein the liquid guide cavity (21) is provided with a first through hole (211) intercommunicating with the liquid storage cavity at an upper part thereof and a second through hole (212) intercommunicating with the detection cavity (19) at a lower part thereof;
a piston (22) sliding along the liquid guide cavity is disposed inside the liquid guide cavity (21), and has an initial state for intercommunicating the liquid guide cavity (21) with the liquid storage cavity (20) and a detection state for intercommunicating the liquid chamber guide cavity (21) with the detection chamber (19);
a third through hole (213) is disposed on a cup wall of the cup body (9) away from the detection chamber (19) and intercommunicated with the liquid guide cavity (21); and the cartridge (17) is disposed vertically inside the detection cavity (19).

15. The reagent cup for semi-quantitative detection of human chorionic gonadotropin according to claim 14, **characterized in that**, a liquid storage tank (221) is disposed on the piston (22); when the piston (22) is in the initial state, the liquid storage tank (221) of the piston is aligned with the first through hole (211), so that the liquid guide cavity (21) is intercommunicated with the liquid storage cavity (20); when the piston (22) is in the detection state, the liquid storage tank (221) of the piston is aligned with the second through hole (212), so that the liquid guide cavity (21) is intercommunicated with the detection cavity (19).

16. The reagent cup for semi-quantitative detection of human chorionic gonadotropin according to claim 14 or 15, **characterized in that**, a boost member (23) for boosting the piston is provided on the cup cover (10), and detachably fitted on the cup cover (10).

17. The reagent cup for semi-quantitative detection of human chorionic gonadotropin according to claim 11 or 12, **characterized in that**, the cartridge (17) is fixed in parallel inside the cup cover (10).

18. The reagent cup for semi-quantitative detection of human chorionic gonadotropin according to claim 17, **characterized in that**, a support body (24) is disposed on an outer edge of the cup cover (10) for fixing the reagent cup placed on its side, and a scale line (25) is arranged on the cup body (9).

19. Use of a test paper for semi-quantitative detection of human chorionic gonadotropin according to any one of claims 1-4, the test paper for semi-quantitative detection of human chorionic gonadotropin prepared by the method according to any one of claims 5-9, or a reagent cup for semi-quantitative detection of human chorionic gonadotropin according to any one of claims 10-18 for detecting human pregnancy cycle, ectopic pregnancy, miscarriage and trophoblastic disease.
